# EUROPEAN PATENT APPLICATION

(11) **EP 4 749 241 A1**
(43) Date of publication of application: **27.05.2026**
(21) Application number: 24215122.3
(22) Date of filing: 25.11.2024
(51) Int. Cl.: G01B 9/02015, G01B 9/02091

(54) **OPTICAL COHERENCE TOMOGRAPHY DEVICE**

(71) Applicant: IT Concepts GmbH, 35633 Lahnau (DE)
(72) Inventor: Gail, Jonathan, 35685 Dillenburg (DE); Boll, Alexander, 35606 Solms (DE); Bahr, Sönke Uwe, 64297 Darmstadt (DE); Neddermeyer, Werner, 6496 Echternach (LU)
(74) Representative: Grupe, Dirk Bernhard Willi

(57) **Abstract**

An Optical Coherence Tomography, OCT, device (100; 200; 410), in particular for an endoscopic measuring system (400), comprises at least one light source (110; 210); at least one cascaded interferometer arrangement (120, 140; 220, 240) having at least a first interferometer (120; 220) configured to receive light (L_S) emitted by the light source (110; 210) and at least a second interferometer (140; 240), the second interferometer (240) arranged behind the first interferometer (120) with respect to an optical path (L_S, L_IL, L_IM) of the OCT device (100; 200; 410); at least one image sensor (180; 280); and; at least one image fiber (160; 260) arranged between the cascaded interferometer arrangement (120, 140; 220, 240) and the image sensor (180; 280) with respect to the optical path (L_S, L_IL, L_IM) of the OCT device (100; 200; 410). The image fiber (160; 260) is configured to receive at least one interferometric image (L_IM) output by the second interferometer (140; 240) and to transmit the interferometric image (L_IM) towards the image sensor (180; 280) for detection of the interferometric image (L_IM) by means of the image sensor (180; 280).

## Description

The disclosure relates to an Optical Coherence Tomography, OCT, device. The disclosure further relates to an optical measuring system and a method of operating an OCT device.

### Background

Endoscopes, such as borescopes, are conventionally used for optical inspection of objects and/or regions with reduced mechanical and optical accessibility. Typical examples include human or animal body parts, such as blood vessels, organs, etc., hollow machine compartments, such as engine interiors, aircraft structures, etc., geological cavities, and the like. Endoscopes facilitate a mechanical and optical access to an interior of such structures, typically, by having a relatively small extension perpendicular to an insertion direction of the endoscope, combined with light capturing facilities, in particular image capturing facilities, which may be suited for various applications, and by allowing an operator to control the endoscope from outside the structure.

Furthermore, optical measuring, including optical coherence tomography, is widespread in industry, research, and other professional and non-professional environments. Typical applications of optical measuring include quality assessment, for example, in the automated production of goods, etc., technical inspection, for example, for determining deformation, abrasion or wear on mechanically exposed machine parts, and the like.

There is a continuous need for improved optical measuring and for improved miniaturization of measuring devices.

### Summary

Accordingly, there is provided an Optical Coherence Tomography, OCT, device according to claim 1, an optical measuring system according to claim 14, and a method according to claim 15.

According to a first aspect, an Optical Coherence Tomography, OCT, device, in particular for an endoscopic measuring system, is provided. The OCT device comprises at least one light source and at least one cascaded interferometer arrangement having at least a first interferometer configured to receive light emitted by the light source and at least a second interferometer, the second interferometer arranged behind the first interferometer with respect to an optical path of the OCT device. The OCT device further comprises at least one image sensor and at least one image fiber arranged between the cascaded interferometer arrangement and the image sensor with respect to the optical path of the OCT device. The image fiber is configured to receive at least one interferometric image output by the second interferometer and to transmit the interferometric image towards the image sensor for detection of the interferometric image by means of the image sensor.

By the aforesaid features, an OCT device as provided herein facilitates that interferometric image information, in particular spatially resolved image information, can be transmitted from the cascaded interferometer arrangement and be acquired, by means of the image fiber and the image sensor. In this way, optical measuring in a spatially extended measuring field is facilitated. Moreover, optical measuring information can be transmitted and acquired, simultaneously, on multiple spatially distinct points of one or more objects in a measuring field of the OCT device. Efficient measuring, in particular on spatially extended objects, can thus be improved.

Furthermore, by providing a (in particular, flexible) image fiber, the image sensor can be flexibly arranged with respect to the second interferometer, and, optionally, with respect to further components of the OCT device. This facilitates an arrangement of the image sensor remote from, and movable relative to, the second interferometer. This facilitates in particular that the second interferometer is arrangeable in a movable measuring head of an optical measuring system, whereas the image sensor is arrangeable external to the measuring head, such as in a stationary base unit of the same optical measuring system. In this way, a size of the movable measuring head is reduceable relative to if a rigid arrangement of the second interferometer and the image sensor was used. Furthermore, by enabling an arrangement of the image sensor external to a measuring head, restrictions concerning an image sensor type, which are normally posed by requirement specifications and/or expected measuring conditions, can be avoided. Such restrictions conventionally include a limitation of a size of the image sensor, or they follow from required tolerances concerning extreme temperatures, wide temperature ranges, rapid temperature variations, high humidity, resistivity to chemicals, shock and vibration, etc., which may result from a limited accessibility, or from physical and/or chemical conditions, at an intended place of measuring.

Furthermore, by providing a cascaded interferometer arrangement, it is enabled that one or more optical settings of the OCT device are implementable in the first interferometer, wherein such optical settings influence an optical situation at the second interferometer, too, as the second interferometer is arranged (optically) behind the first interferometer. In this way, a measuring process by means of the OCT device can be optically configured, in particular controlled, for example, by selecting and/or varying the one or more optical settings, remote from the second interferometer. In this way, a size of a measuring head comprising the second interferometer is reduceable relative to if means for implementing such optical settings needed to be arranged in the measuring head, as could otherwise be the case if a single-interferometer setup was used. Furthermore, in this way, it is avoidable that advantages in terms of mobility which are enabled by means of the image fiber between the interferometer arrangement and the image sensor, as set out above, are partially or totally annihilated by an increased size and/or weight of the interferometer setup, for enabling such optical settings, in a mobile portion of the OCT device which may have to be brought into proximity of an intended place of measuring. This is achieved, in particular, by combined provision of the image fiber and a cascaded interferometer arrangement of the OCT device.

Furthermore, by enabling an implementation of optical settings of the OCT device in the first interferometer, the disclosure also enables that one or more variable optical settings of the OCT device are implementable, and controllable, external to a measuring head of an optical measuring system. Restrictions regarding a variation range of the setting(s) and/or an actuator type for varying the setting(s), which are normally posed by requirement specifications and/or expected measuring conditions, such as limited space, extreme temperatures, temperature ranges, temperature variations, humidity, chemicals, shock and vibration, etc., at an intended place of measuring, can be avoided.

The disclosure is based on the inventors' finding that, contrary to a widespread assumption in the field of optical devices, interferometric image information as it is obtainable by means of an interferometer arrangement, including a cascaded interferometer arrangement, is usable for transmission by means of an image fiber, and for acquisition by means of an image sensor, while maintaining optical coherence at an extent suitable for interferometric measuring applications, in particular spatially resolved interferometric measuring, in various measuring scenarios, including measuring scenarios in which limited accessibility precludes a use of tactile measuring tools and short-distance microscopes.

A possible explanation for the identified suitability of a transmission of interferometric image information via an image fiber, especially the maintaining of optical coherence in a (multi-core) image fiber, in typical Optical Coherence Tomography measuring scenarios, could be that each core of the multi-core image fiber effectively functions in these cases, sufficiently well for determining a relative interference intensity, as a (quasi-)mono-mode fiber for a corresponding portion of the interferometric image, thus preserving coherence in each core and, accordingly, also when the interferometric image is projected onto the image sensor.

In the context of the present disclosure, the term "interferometer", unless further specified, can denote any optical setup that enables generation and controlled and/or controllable use of optical interference, especially, for the purpose of optical measuring. Various types of interferometers are known, such as Michelson interferometers, Mach-Zehnder interferometers, Fabry-Pérot interferometers, etc. In the context of the present disclosure, the term "interferometer" is not meant to be restricted to any specific one(s) of such known types or others but may denote any interferometer setup suited for the purposes set forth herein.

The light source can comprise a white-light source. In addition, or as an alternative, the light source can comprise one or more light emitting diodes, LED, in particular one or more surface-mounted device-, SMD-, LEDs. Using one or more SMD LEDs as the light source facilitates a high illumination efficiency in a compact arrangement of the OCT device. In the context of the present disclosure, the term "white light", unless further specified, can denote any light having an extended spectral width, with a continuous and/or a discrete distribution of its spectral components over at least a portion of the spectral width, and wherein at least some of the spectral components are non-coherent relative to each other, such that a coherence length of the light is essentially limited.

The second interferometer can be configured to be arranged in a measuring head of an optical measuring system, in particular of an endoscopic measuring system. In addition, or as an alternative, the image sensor can be configured to be arranged external to the measuring head, in particular in a base unit of the optical measuring system. The image fiber can be part of a flexible connection between the measuring head and the image sensor.

In every plane perpendicular to an insertion direction of the measuring head, the measuring head can have a largest diameter in a range between 5 and 50 Millimeters, preferably between 6 and 20 Millimeters, more preferably between 7 and 10 Millimeters.

The OCT device can further comprise a flexible illumination fiber arranged between the first interferometer and the second interferometer. The illumination fiber can comprise a single-mode fiber.

By means of the flexible illumination fiber, the first interferometer, the light source, and any other components of the OCT device arranged before the second interferometer with respect to the optical path of the OCT device can be flexibly arranged with respect to the second interferometer. This facilitates an arrangement of the second interferometer remote from, and movable relative to, the first interferometer and any other components of the OCT device arranged optically before the second interferometer. This facilitates in particular that the second interferometer is arrangeable in a movable measuring head of an optical measuring system, whereas the light source, the first interferometer and any other components of the OCT device arranged optically before the second interferometer are arrangeable external to the measuring head and external to a movable probing tool, such as an endoscope of the optical measuring system, comprising the measuring head.

The light source, the first interferometer and, optionally, any other components of the OCT device arranged optically before the second interferometer can be arrangeable external to a measuring head of an optical measuring system, in particular in a base unit of the optical measuring system.

In this way, a size of the movable measuring head and/or a size of the probing tool is further reduceable, especially relative to if a rigid arrangement of the first interferometer, the second interferometer, and, potentially, the light source and/or any other components of the OCT device arranged optically before the second interferometer, was used.

Furthermore, by enabling an arrangement of the first interferometer, the light source, and any other components of the OCT device arranged optically before the second interferometer external to a measuring head and external to a movable probing tool comprising the measuring head, restrictions concerning a size and/or a weight of the probing tool, which are normally posed by requirement specifications and/or expected measuring conditions, can be avoided.

The first interferometer can be further configured to split light received from the light source into at least one primary beam and at least one first reference beam, and to output the primary beam and the first reference beam towards the second interferometer. The first interferometer can be configured to produce an optical offset between the first primary beam and the first reference beam as output towards the second interferometer.

The optical offset can comprise a spatial, a temporal and/or a phase optical offset. In addition, or as an alternative, the light source and the first interferometer can be configured such that the optical offset exceeds a coherence length of the light received by the first interferometer from the light source.

The optical offset produceable by means of the first interferometer can be variable. In addition, the OCT device can further comprise at least one actuator for varying a position of at least one optical component of the first interferometer for varying the optical offset. Varying the position of the at least one optical component can cause variation of an optical path length of the first reference beam in the first interferometer. The first interferometer can be configured to vary the variable optical offset at least partially by varying the optical path length of the first reference beam.

The variable optical offset can be variable in accordance with a measuring range of the OCT device.

In addition, or as an alternative, varying the position of the at least one optical component can comprise varying the position of a mirror to change a geometric length changing an optical density of at least a portion of at least one optical path in the first interferometer by varying a position

The second interferometer can be further configured to split light received from the first interferometer into at least one measuring beam and at least one second reference beam; output the measuring beam towards a measuring field of the OCT device; receive at least one object beam, the object beam generated by an at least partial reflection of the measuring beam at an object in the measuring field of the OCT device; and, output the second reference beam and the object beam towards the image fiber.

The interferometric image can be indicative of a depth of the object based on a light intensity detectable by means of the image sensor. The light intensity can be produced by optical interference in accordance with the optical offset and the depth of the object.

The optical interference can comprise an interference between light of one of the primary beam and the first reference beam respectively contained in the second reference beam and light of the respective other one of the primary beam and the first reference beam respectively contained in the object beam.

Each one of the second reference beam and the measuring beam output by the second interferometer can have a spatially, in particular linearly or planarly, extended cross section for producing a spatially extended interferometric image. In addition, the image sensor can be configured to provide spatially resolved detection of a light intensity in the interferometric image. Thereby, spatially extended measuring of a depth profile of an object in the measuring field of the OCT device can be enabled.

According to another aspect, an optical measuring system, in particular an endoscopic measuring system, is provided. The optical measuring system comprises at least one OCT device as provided herein; at least one measuring head; and, at least one base unit. The first interferometer, the light source and the image sensor of the OCT device are arranged in the base unit. The second interferometer of the OCT device is arranged in the measuring head.

The optical measuring system can comprise a probing tool in the form of a borescope, and the measuring head can be a measuring head of the borescope. In addition, or as an alternative, the optical measuring system can comprise one or more additional image acquisition means, in particular at least one camera, for enabling acquisition of photographic image information about an environment of the measuring field of the OCT device and/or about one or more objects in the measuring field of the PCT device. The photographic image information can be configured to serve as guidance for a user when positioning the OCT device relative to an object of interest.

According to another aspect, a method of operating an optical coherence tomography, OCT, device, in particular for an endoscopic measuring system, is provided. The OCT device comprises at least one light source; at least one cascaded interferometer arrangement having at least a first interferometer configured to receive light emitted by the light source and at least a second interferometer, the second interferometer arranged behind the first interferometer with respect to an optical path of the OCT device; at least one image sensor; and, at least one image fiber arranged between the cascaded interferometer arrangement and the image sensor with respect to the optical path of the OCT device. The image fiber is configured to receive at least one interferometric image output by the second interferometer and to transmit the interferometric image towards the image sensor for detection of the interferometric image by means of the image sensor. The method comprises outputting, by means of a control unit and towards at least one actuator of the OCT device, a control signal for varying an optical offset between a primary beam and a first reference beam as output by the first interferometer of the OCT device; receiving, by means of the control unit and from the image sensor of the OCT device, image data of at least one interferometric image for each of various optical offsets; analyzing, by means of the control unit, the image data of the plurality of interferometric images to determine a variable light intensity caused by variable optical interference in accordance with the various optical offsets; and, determining, by means of the control unit, at least one depth of at least one object in the measuring field of the OCT device based on the determined variable light intensity.

### Brief Description of the Drawings

Further details, objects and advantages of the disclosure will be apparent from the detailed description and the drawings. There is shown in:
- Figs. 1 and 2: Optical Coherence Tomography, OCT, devices according to various embodiments;
- Fig. 3: detected interference during a depth scan according to an example;
- Fig. 4: an optical measuring system according to an embodiment; and,
- Fig. 5: a method of operating an OCT device according to an embodiment.

### Detailed Description

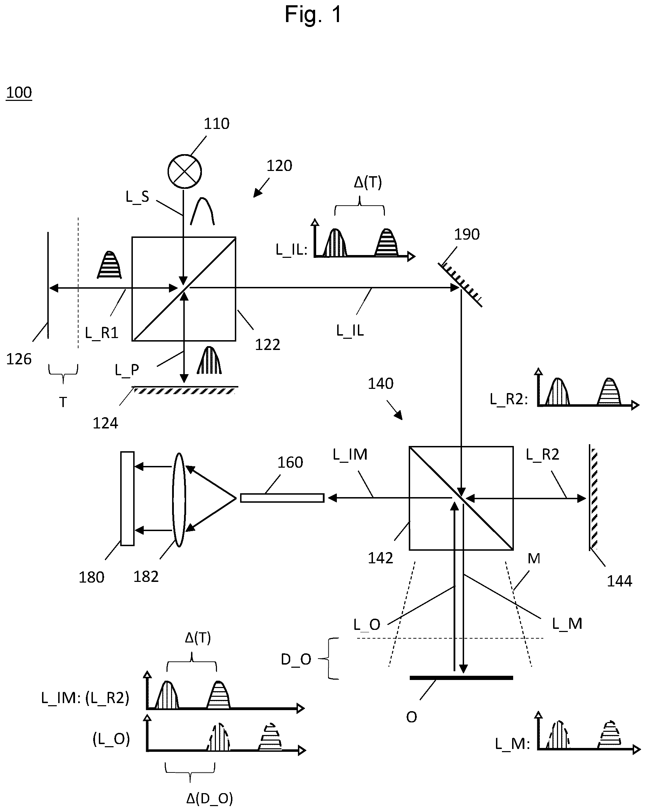

Fig. 1 shows schematically and exemplarily an optical coherence tomography, OCT, device 100. The OCT device 100 comprises a light source 110, a first interferometer 120, a second interferometer 140, an image fiber 160, and an image sensor 180. The aforesaid components are arranged along an optical path of the OCT device 100, as indicated in Fig. 1 by the single-pointed arrows between these components and as described in more detail below. In particular, the first interferometer 120 and the second interferometer 140 constitute a cascaded interferometer arrangement of the OCT device 100.

The OCT device 100 is configured to enable the determining of a depth D_O of an object O positioned in a measuring field M of the OCT device 100 using interferometric image information, as illustrated in Fig. 1 and as described in more detail below.

During an operation of the OCT device 100, the light source 110 outputs a source beam L_S of light towards the first interferometer 120. As illustrated schematically in Fig. 1 by the shape of a positive envelope of a wave package next to the source beam L_S, the light output by the light source 110 exhibits a limited coherence length. For this purpose, the light source 110 comprises in some examples a white light source having a continuous spectral range, thereby causing a correspondingly limited coherence length.

The source beam L_S is received at the first interferometer 120. The first interferometer 120 comprises a first beam splitter 122. In the shown example, the first beam splitter 122 is a cubic beam splitter, for convenience of illustration. However, in other examples of the OCT device 100, other types of beam splitter, such as beam splitting discs, or others, are used. The first beam splitter 122 splits the received source beam L_S into a primary beam L_P and a first reference beam L_R1. The primary beam L_P is output by the first beam splitter 122 towards a first fixed mirror 124 of the first interferometer 120, and it is reflected at the first fixed mirror 124 towards the first beam splitter 122. The first reference beam L_R1 is output by the first beam splitter 122 towards a reference mirror 126 of the first interferometer 120, and it is reflected at the reference mirror 126 towards the first beam splitter 122. The reflected primary beam L_P and the reflected first reference beam L_R1 are, at least partially, recombined, by superposition at the first beam splitter 122, and output from the first beam splitter 122, as an illumination beam L_IL, towards the second interferometer 140.

The first interferometer 120 is configured to create an optical offset, Δ(T), in a propagation direction of the illumination beam L_IL, between the recombined primary beam L_P and first reference beam L_R1 as they are output towards the second interferometer 140. For this purpose, the first interferometer 120 is configured such that, between the splitting of the source beam L_S and the recombining of the primary beam L_P and of the first reference beam L_R1, an optical path length of the primary beam L_P is different from an optical path length of the first reference beam L_R1. In the example shown in Fig. 1, the difference is indicated by a length T by which a geometric distance of the reference mirror 126 from the first beam splitter 122 exceeds a geometric distance of the first fixed mirror 124 from the first beam splitter 122. Due to the length T, light in the first reference beam L_R1 propagates longer than light in the primary beam L_P and, hence, is spatially and temporally offset relative to light which propagates in the primary beam L_P when the beams are recombined.

The offset Δ(T) is schematically illustrated in Fig. 1 by fractional wave packages next to each of the primary beam L_P, the first reference beam L_R1 and the illumination beam L_IL. Each of the fractional wave packages shown next to the primary beam L_P and the first reference beam L_R1 corresponds to a fraction of the wave package shown next to the source beam L_S, after splitting the source beam L_S at the first beam splitter 122. The fractional wave packages next to the illumination beam L_IL show the recombined fractional wave packages from the primary beam L_P and the first reference beam L_R1 plotted over time. As shown, in the recombined beams constituting the illumination beam L_IL, the fractional wave package of the primary beam L_P precedes the fractional wave package of the first reference beam L_R1 by an optical offset Δ(T), in accordance with a twofold passage of the distance T by the first reference beam L_R1 divided by the propagation velocity of the first reference beam L_R1 in the first interferometer 122. As further shown, the optical offset Δ(T) is selected to exceed a coherence length of the light provided in the source beam L_S.

At the second interferometer 140, the received illumination beam L_IL is split at a second beam splitter 142 into a second reference beam L_R2 and a measuring beam L_M. The second reference beam L_R2 is output by the second beam splitter 142 towards a second fixed mirror 144, and it is reflected at the second fixed mirror 144 towards the second beam splitter 142. The measuring beam L_M is output by the second beam splitter 142 towards the measuring field M. When an object O is positioned in the measuring field M, a portion of the measuring beam L_M is reflected at the object O towards the second beam splitter 142, thus constituting an object beam L_O. Analogously to the situation at the first interferometer 120, the reflected second reference beam L_R2 and object beam L_O are at least partially recombined by superposition at the second beam splitter 142 and output, as an image beam L_IM, towards the image fiber 160.

As schematically shown in Fig. 1 by fractional wave packages next to the second reference beam L_R2, the measuring beam L_M and the image beam L_IM, each of the second reference beam L_R2 and the measuring beam L_M contains a fraction of the illumination beam I_IL. Moreover, after combination of the object beam L_0 and the reflected second reference beam L_R2 at the second beam splitter 142, corresponding fractions of the illumination beam L_IL are contained, and combined, in the image beam L_IM. Furthermore, the fractional wave packages of the illumination beam L_IL as contained in the second reference beam L_R2 and the object beam L_O are offset relative to each other when combined to the image beam L_IM, in accordance with a difference of the optical path length of the output and reflected second reference beam on one side and the combination of the output measuring beam L_M and the received object beam L_O on the other side, analogously to what has been described above in connection with the first interferometer 120.

In the shown example, a geometric distance of the object O from the second beam splitter 142 exceeds a geometric distance of the second fixed mirror 144 from the second beam splitter 142 by a length D_0. Accordingly, the fractional wave packages of the illumination beam L_IL as contained in the image beam L_IM provided by the object beam L_O are relatively delayed in comparison to the fractional wave packages of the illumination beam L_IL as contained in the image beam L_IM provided by the second reference beam L_R2 by an optical offset Δ(D_0).

In the shown scenario, the offset Δ(D_0) resulting from the specific distance of the object O from the second beam splitter 142, according to a specific depth of the object O, equals the optical offset Δ(T) resulting from the length T in the first interferometer 120. In this case, the fraction of light from the primary beam L_P as contained in the object beam L_0 coincides in the image beam L_IM with the fraction of light from the first reference beam L_R1 as contained in the second reference beam L_R2. Since all fractional wave packages are produced based on the same wave package initially contained in the source beam L_S, any coinciding ones of these fractional wave packages interfere in the image beam L_IM. Conversely, light portions coinciding in the image beam L_IM which do not derive from the same portion of light in the source beam L_S, for example, if the offset Δ(D_0) differs from the offset Δ(T) by more than the coherence length of the light provided by the light source 110, will not cause detectable interference in the image beam L_IM. In this way, when the offset Δ(T) is known, an interference image contained in the image beam L_IM enables determining whether the object O, or at least portions thereof, is at a specific depth with respect to the OCT device 100, which corresponds optically to the distance T, or not.

After transmission by the image fiber 160, the image beam L_IM is out-coupled from the image fiber 160 towards the image sensor 180. In the shown example, an optical component, such as a lens 182, is arranged between the image fiber 160 and the image sensor 180 for projection of the interference image onto a detection plane of the image sensor 180.

The image sensor 180 enables detection of the interference image and generates a corresponding sensor signal indicative of the detected image information. The sensor signal, which is output by the image sensor 180, thus is available for further processing, including automatic determining of a depth of the object O based on the interference information encoded in the interference image, as described herein.

In the shown example, the OCT device 100 further comprises a fixed deflection mirror 190 which deflects the illumination beam L_IL in the direction of the second interferometer 140. Other examples of the OCT device 100 do not comprise a deflection mirror 190. In some of these examples, the second beam splitter 142 is arranged in alignment with the output direction of the illumination beam L_IL from the first beam splitter 122, hence no deflection of the illumination beam L_IL is needed. In other examples, additional or other means than the deflection mirror 190 are used for directing the illumination beam L_IL output by the first interferometer 122 towards the second interferometer 140, such as one or more optical fibers.

In some examples, provision of the image fiber 160 and of the image sensor 180 enables the transmission and detection of spatially, in particular linearly or planarly, extended interferometric image information. Thus, when a measuring beam L_M having an extended beam profile is used, for example, by providing a spatially extended illumination beam L_IL, a spatially extended surface of the object O will be illuminated, and, by superposition of correspondingly extended object beam L_0 and second reference beam L_R2, a spatially extended image beam L_IM is produced. This allows for spatially resolved interference information contained in the interference image. Thus, for example, depth information can be retrieved from the acquired image sensor data for different ones of multiple surface portions of the object O.

Additionally, when the image fiber 160 is flexible, provision of the image fiber 160 enables mechanically decoupling a position and orientation of the image sensor 180 from the position and orientation of the second interferometer 140. In this way, an arrangement of the second interferometer 140 in a movable probing tool, such as an endoscopic, is facilitated, wherein the image sensor 180 does not need to be arranged in the same endoscope, but, for example, can be arranged in a base unit of an optical measuring system comprising the endoscope. Thus, an application range of the optical coherence tomography device 100 is extended, especially for applications in measuring scenarios that demand easy mobility and/or reduced size or weight of the probing tool.

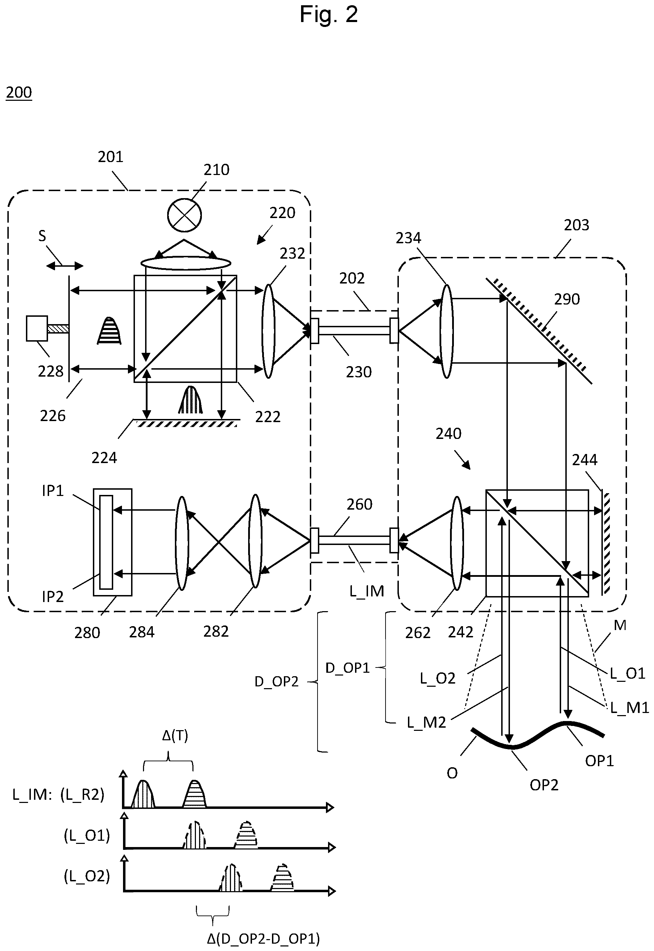

For example, as set forth in connection with Fig. 2 below, in some implementations the second interferometer 140 is configured to be arranged in a measuring head of an optical measuring system, such as an endoscopic measuring system. Additionally, or alternatively, in some examples, the first interferometer 120 and the image sensor 180 are configured to be arranged external to the measuring head, such as in a base unit of the optical measuring system.

Arranging the first interferometer in a base unit of an optical measuring system while enabling mobility of a measuring head comprising the second interferometer 142 is facilitated in some of the aforesaid examples by provision of a flexible illumination fiber instead of, or in addition to, a rigid arrangement of the first and second interferometers 120, 140, or deflection mirror 190, for guiding the illumination beam L_IL from the first interferometer 120 towards the second interferometer 140.

Furthermore, as the reference mirror 126 is arranged in the first interferometer 120 and its position determines the optical offset Δ(T), a measuring range, such as a detectable depth range, of the OCT device 100 is configurable external to the second interferometer 140. Thus, the second interferometer, including, for example, its arrangement in a measuring head of a probing tool, does not need to comply with structural requirements, corresponding to geometric length T or others, imposed by an intended measuring range. This further facilitates a miniaturized implementation of a measuring head for use with the OCT device 100.

In some examples, a position of the reference mirror 126 is fixed. In such examples, accordingly, the optical offset Δ(T) is likewise fixed. Such examples of the OCT device 100 are suited for checking, based on an occurrence of interference in the interferometric images, whether or not one or more portions of an object O are positioned in the measuring field M at the corresponding specific depth D_0. In other examples, a position of the reference mirror 126 is selectable. Such examples of the OCT device 100 are suited for selecting the corresponding depth at which presence of an object O in the measuring field M is detectable, based on a corresponding occurrence of interference in the interferometric image information.

In other examples of the OCT device 100, as described in more detail below in connection with Fig. 2, a position of the first reference mirror 126 is dynamically variable, for example, along a predetermined mechanical travel path, or: scanning path. In combination with a simultaneous acquisition of multiple interferometric images for successive positions of the first reference mirror 126, a depth scan can be performed along a depth direction of the measuring field M. This allows, for example, determining a depth profile of an extended surface of an object O in the measuring field M, especially when performed in combination with the acquisition of spatially extended image information by means of the image sensor 180.

In some examples, in which the OCT device 100 is configured for use in an endoscopic measuring system, a minimal measurable depth of an object O in the measuring field M is less than 10 Millimeters, such as less than 5 Millimeters, and a maximal measurable depth is more than 100 Millimeters, such as more than 200 Millimeters. In some examples, in which the position of the reference mirror 126 is dynamically variable, this corresponds to a length of the scanning path of the reference mirror 126 larger than 90 Millimeters, such as larger than 195 Millimeters. In other examples, in which the position of the reference mirror 126 is dynamically variable, a length of the scanning path of the reference mirror 126 is smaller than 20 Millimeters, such as smaller than 10 Millimeters. A shorter length of the scanning path is particularly suited for measurements on relatively even surfaces, as in such cases a scan, corresponding to a measurement, can be performed in less time, thus reducing an optical impact on the measurement result which may be caused by vibration or other movements of the OCT device relative to the measured surface during the scan.

Fig. 2 shows schematically and exemplarily an optical coherence tomography, OCT, device 200 according to another example.

The OCT was 200 comprises a light source 210, a first interferometer 220, a second interferometer 240, an image sensor 280, and a deflection mirror 290. The first interferometer 220 comprises a first beam splitter 222, a first fixed mirror 224 and a first reference mirror 226. The second interferometer 240 comprises a second beam splitter 242, a second reference mirror 244, and it is configured to output at least one measuring beam L_M1, L_M2 towards a measuring field M of the OCT device 200, and to output one or more received object beams L_O1, L_O2 and a reflected second reference beam towards the image fiber 260 for transmission of interferometric image information towards the image sensor 280. Concerning these features of the OCT device 200, the description of the corresponding features of the OCT device 100 applies analogously unless otherwise clear from Fig. 2 and the following description.

As indicated in Fig. 2 by the paired parallel arrows, which represent parallel light paths, the OCT device 200 is configured to be operated with light beams having a spatial, such as linearly or planarly, extended cross-section, or: beam profile, to allow spatially resolved depth measurements on an object O, as described above. In other examples, a spatially extended distribution of light in a cross-sectional plane of one or more of the beams is obtainable by the provision of multiple light sources 110 arranged adjacent each other, beam expansion means, or the like.

The OCT device 200 further comprises an actuator 228 as part of the first interferometer 120. The actuator 228 is configured to vary the position of the first reference mirror 226 parallel to a propagation direction of the first reference beam L_R1. As indicated in Fig. 2 by the double arrow above the first reference mirror 226, the actuator 228 is configured to vary the position of the first reference mirror 226 reversibly over a scan length S. In this way, by operation of the actuator 228, an optical offset Δ(T) induced by the position of the first reference mirror 226 can be selected prior to a measuring process, for example, in accordance with a specific depth D_OP1, D_OP2 of interest to the user. In addition, or in other examples, the optical offset Δ(T) thus can be dynamically varied during a measuring process, for example, for performing a depth scan along a depth direction of the measuring field M, as described above.

The OCT device 200 further comprises a flexible illumination fiber 230 for directing the illumination beam output by the first interferometer 230 towards the second interferometer 240. In addition, the OCT device 200 comprises an illumination fiber in-coupling lens 232 for coupling light into the illumination fiber 230, and an illumination fiber out-coupling lens 234 for re-collimating light which has been out coupled from the illumination fiber 230.

The illumination fiber 230 and the image fiber 260 constitute a flexible connection between a first OCT device components group 201 of the OCT device 200 on one side of the flexible connection provided by the illumination fiber 230 and the image fiber 260, which themselves constitute a second OCT device components group 202, and a third OCT device components group 203 on another side of that flexible connection. In the shown example, the first OCT device components group 201 comprises the light source 210, the first interferometer 220, and the image sensor 280 with imaging optics 282, 284. Moreover, in the shown example, the third OCT device components group 203 comprises the second interferometer 240 and the deflection mirror 290.

In some examples, the first OCT device components group 201 is configured to be arranged in a stationary base unit of an optical measuring system, whereas the third OCT device components group 203 is configured to be arranged in a mobile probing tool of the same optical measuring system, the mobile probing tool including a measuring head of the optical measuring system. In such examples, the second OCT device components group 202 provides a flexible connection between the base unit and the probing tool.

In the shown example, the object O comprises various object portions OP1, OP2, which constitute an uneven surface of the object O. As can be seen in Fig. 2, the different object portions OP1, OP2 thereby have different depths D_OP1, D_OP2 relative to the OCT device 200. The different depths of the object portions OP1, OP2 cause different optical path lengths for different cross-sectional portions L_M1, L_M2 of the measuring beam impinging on the object O and for their respective reflections as contained in different cross-sectional portions L_01, L_02 of the object beam. Consequently, as described above, interference images which are transmitted by the image fiber 260 and detected by means of the image sensor 280 will exhibit interference in image regions corresponding to any of the object portions OP1, OP2 at respectively different positions of the first reference mirror 226 along the scanning path S, for example, when a depth scan is performed by means of the OCT device 200.

This is schematically illustrated at the bottom of Fig. 2 by fractional wave packages which are contained in the image beam L_IM. The fractional wave packages provided by the second reference beam constitute the reference for determining a depth, by the optical offset Δ(T). In the example, fractional wave packages contained in the image beam L_IM and provided by the first portion L_01 of the object beam or from the second portion L_02 of the object beam are relatively delayed with respect to the fractional wave packages provided by the second reference beam L_R2. Furthermore, fractional wave packages provided by the second portion L_02 of the object beam are relatively delayed with respect to the fractional wave packages provided by the first portion L_01 of the object beam, corresponding to the larger depth D_OP2 of the second object portion OP2 in comparison to the depth D_OP1 of first object portion OP1.

For the shown position of the first reference mirror 226, the relative depth D_OP1 of the first object portion OP1 with respect to the second reference beam L_R2 corresponds to the optical offset Δ(T) induced by the position of the first reference mirror 226. In consequence, light from the first reference beam as contained in the second reference beam L_R2 interferes with light from the primary beam as contained in the first portion L_01 of the object beam corresponding to the first object portion OP1 in the image beam L_IM. Thus, interference will be detectable by means of the image sensor 280 at a first image region IP1 of the image sensor corresponding to an image region on which the first object portion OP1 is projected. In contrast, no interference occurs with light contained in the second portion L_02 of the object beam corresponding to the second object portion OP2.

Continuing with the same example, when a depth scan is performed towards larger depths, by scanning the first reference mirror 226 towards longer path lengths of the first reference beam, at a certain position of the first reference mirror 226 the optical offset Δ(T) will have increased to such an extent that light from the first reference beam as contained in the second reference beam L_R2 interferes with light from the primary beam as contained in the second portion L_02 of the object beam corresponding to the second object portion OP2 in the image beam L_IM. At that instance, interference will be detectable at a second image region IP2 of the image sensor 280 on which the second object portion OP2 is projected, whereas no interference occurs with light corresponding to the first object portion OP1.

In the above manner, a spatially resolved depth profile of the object O can be determined, especially be built up, by combining the depth information obtained for individual ones of various object portions OP1, OP2 from different ones of the acquired interferential images.

In the above examples, for convenience of illustration, some of the mirrors of the OCT devices 100, 200 have been described as fixed. It will be understood, nonetheless, that some, or all, of such mirrors can be movable in some examples while still enabling some, or all, of the aforesaid advantages. Furthermore, in the above examples, for convenience of illustration, specific ones of the mirrors of the OCT devices 100, 200 have been described as movable for specific purposes, and the path length of specific ones of the beams in one or more of the interferometers have been described as exceeding the path length of other ones of the beams by specific amounts. It will be understood, nonetheless, that for achieving the described effects other ones of the mirrors can be chosen to be movable instead, and at least some of said specific ones of the beams in one or more of the interferometers can have a path length which falls short off, instead of exceeds, a path length of at least some of said other ones of the beams by correspondingly specific lengths, while still enabling some, or all, of the aforesaid advantages. For example, in some implementations the path length of the first reference beam falls short off, instead of exceeds, the path length of the primary beam for producing an optical offset, etc.

Furthermore, for convenience of illustration, in Figs. 1 and 2 various optical components of an OCT device, including mirrors, are schematically shown as having a planar profile. It will be understood, nonetheless, that some, or all, of such components can have a non-planar shape in some examples. Optical components having non-planar shapes, such as curved mirrors, are provided in some examples for optical purposes such as beam guiding, beam shaping, focusing and/or defocusing, as convenient in each individual case. Moreover, in some examples, one or more of the mirrors of the interferometers are implemented as (triple) corner reflectors. This facilitates in some examples an easy installation of the mirrors during manufacture of the OCT device, and/or provides for increased vibration tolerance of the manufactured OCT device, due to the increased optical tolerance provided by such reflectors with respect to potential mechanical misalignment.

Furthermore, in some examples, additional optical components are provided, such as optical polarizers arranged in some, or all, of the optical beams in one or more of the interferometers, combined with Lambda/2- and/or Lambda/4-plates, as required, to compensate for phase flips at beam reflections. The provision of optical polarizers is suited in some examples to reduce undesired optical reflections in the obtained image information.

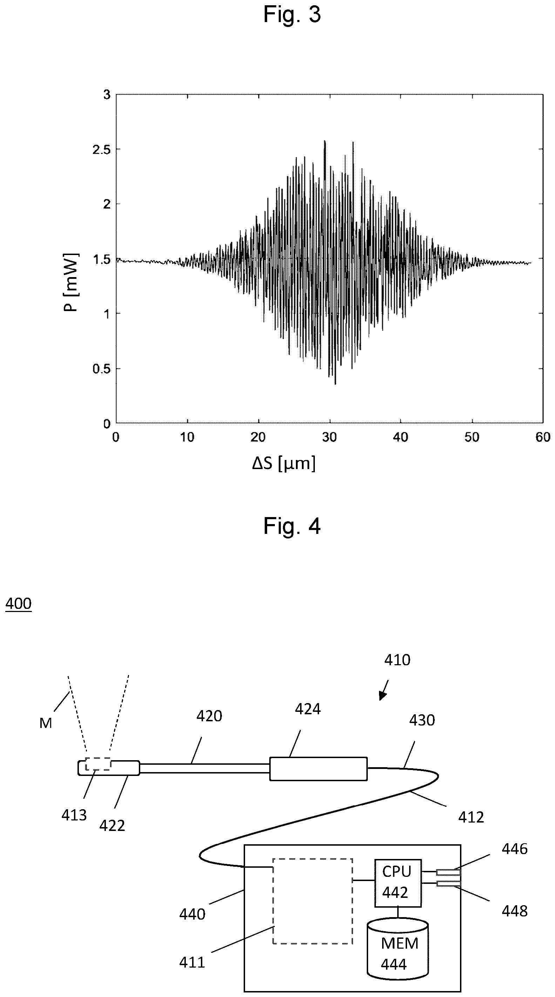

Fig. 3 shows an example of detected interference during a depth scan of an OCT device, such as OCT device 200 described above, with an object placed in the measuring field of the OCT device.

The horizontal axis of the diagram corresponds to a displacement ΔS, measured in Micrometers, of the first reference mirror during the depth scan relative to a start position. The vertical axis of the diagram corresponds to a detected light intensity, measured in Milliwatts, at the image sensor, after subtraction of a background intensity corresponding to an intensity of the image beam in the absence of interference. The subtracted background intensity equals the sum of the intensities of the non-interfering second reference beam and object beam as contained in the image beam.

As can be seen in Fig. 3, detectable interference sets in when the fractional wave packages from the second reference beam and from the object beam begin to coincide. As described above, this happens when, due to the varying position of the first reference beam, a difference between the optical offset ΔT and the offset induced by the depth D_O of the object becomes less than the coherence length of the light provided in the source beam. Correspondingly, the detected interference vanishes again when, due to the continuing motion of the first reference beam in the scanning direction, the difference between the optical offset ΔT and the offset induced by the depth D_O becomes larger than the coherence length of the light provided in the source beam. Between these instances, the detected interference reaches a maximum when the difference between the optical offset ΔT and the offset induced by the depth D_O becomes minimal, such as zero. In that instance, an overlap between the fractional wave packages from the second reference beam and from the object beam as contained in the image beam is maximal. From the known displacement ΔS of the first reference mirror in that instance, the depth D_O thus can be determined.

Fig. 4 shows schematically and exemplarily an optical measuring system 400. The optical measuring system 400 comprises an OCT device 410 according to one or more of the examples described herein. In the shown example, the optical measuring system 400 is an endoscopic measuring system comprising, as a probing tool, an endoscope 420. The endoscope 420 is operatively coupled to a base unit 440 of the optical measuring system 400 by means of a flexible cable 430. In some examples, the base unit 440 is a stationary base unit, and the endoscope 420 is coupled to the stationary base unit 440 by means of the flexible cable 430 to enable movement of the endoscope 420 relative to the base unit 440, for example, by an operator of the optical measuring system 400, while ensuring operative coupling of functionalities of the OCT device 410 which are arranged in the endoscope 420 to functionalities of the OCT device 410 which are arranged in the base unit 440, as described in more detail below.

The endoscope 420 comprises a measuring head 422 and a handle 424. The handle 424 is connected to the measuring head 422 and is configured for manipulating a position of the endoscope 420.

The OCT device 410 comprises multiple OCT device components groups 411, 412, 413. The OCT device components groups 411, 412, 413 correspond, in some examples, to the OCT device components groups 210, 22, 203, respectively, of the OCT device 200. The first OCT device components group 411 comprises components of the OCT device 410 which are arranged in the base unit 240. The second OCT device components group 412 comprises components of the OCT device 410 which are arranged essentially in the one or more cables 412 connecting the endoscope 420 to the base unit 240, such as a flexible illumination fiber and/or a flexible image fiber of the OCT device 410 in accordance with the above examples. The third OCT device components group 413 comprises components of the OCT device 410 which are arranged in the measuring head 422 of the endoscope 420.

As indicated in Fig. 4 by dotted lines, a measuring field M of the OCT device 410 extends from the measuring head 422. In some examples, the third OCT device components group 413 comprises a second interferometer of the OCT device 410.

In some examples, the first OCT device components group 411 comprises each of the first interferometer, a light source and an image sensor of the OCT device 410, in accordance with the above examples.

The optical measuring system 400 further comprises a data processor 442 arranged in the base unit 440 and operatively coupled to the first OCT device components group 411. In addition, the data processor 442 is operatively coupled to a data storage device 444 and to each of an input interface 446 and an output interface 448 of the base unit 440.

The data processor 442 is configured to receive image data which has been obtained by the image sensor of the OCT device 410, and to output control signals for operating the image sensor. The data processor 442 is further configured to process the received image data for determining at least one depth of an object (not shown) in the measuring field M, based on optical interference derivable from at least one interferometric image contained in the image data, in accordance with any of the examples described herein. The data processor 442 is in some examples configured to output the determined depth to the output interface 448 of the base unit 440, for example, for external storage, remote communication, or visual display of a graphical representation of the determined depth profile by means of a display device (not shown), and/or to store the determined depth in the data storage device 444 for later use.

In some examples, operational information for operating the OCT device 410 is stored in the data storage device 444. Operational information of such type includes, in some examples, one or more scan control data for operating one or more actuators of the OCT device 410 for varying an optical offset of the OCT device 410, as described herein.

In some examples, the data storage device 444 contains executable program code which is accessible, and executable, by means of the data processor 442 to configure the data processor 442 for executing one or more of the methods described herein of operating the OCT device 410.

In some examples, the input interface 446 is provided for providing the base unit 440 with updated data concerning operation of the OCT device 410, which in some examples will be stored in the data processing device 444 and/or will be executed in real time by means of the data processor 442 during an operation of the OCT device 410. In other examples, additional and/or other operational data is provided to the data processor 442 via the input interface 446.

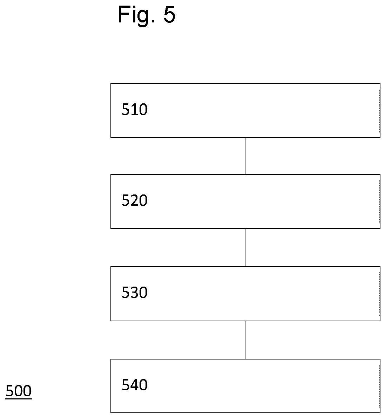

Fig. 5 shows a flow diagram of a method 500 of operating an OCT device. The method 500 is executable using an OCT device in accordance with one or more of the examples described herein, which is operatively coupled to a control unit, such as data processor 442.

The method 500 comprises outputting, by means of the control unit and towards at least one actuator of the OCT device, a control signal for varying an optical offset between a primary beam and a first reference beam as output by a first interferometer of the OCT device, step 510.

The method 500 further comprises receiving, by means of the control unit and from the image sensor of the OCT device, image data of at least one interferometric image for each of various optical offsets, step 520.

The method 500 further comprises analyzing, by means of the control unit, the image data of the plurality of interferometric images to determine a variable light intensity caused by variable optical interference in accordance with the various optical offsets, step 530.

The method 500 further comprises determining, by means of the control unit, at least one depth of at least one object in the measuring field of the OCT device based on the determined variable light intensity, step 540.

In some examples of the method 500, determining the at least one depth based on the determined variable light intensity is performed in accordance with a calibration rule for the individual OCT device.

## Claims

1. Optical Coherence Tomography, OCT, device (100; 200; 410), in particular for an endoscopic measuring system (400), comprising:
at least one light source (110; 210);
at least one cascaded interferometer arrangement (120, 140; 220, 240) having at least a first interferometer (120; 220) configured to receive light (L_S) emitted by the light source (110; 210) and at least a second interferometer (140; 240), the second interferometer (240) arranged behind the first interferometer (120) with respect to an optical path (L_S, L_IL, L_IM) of the OCT device (100; 200; 410),
at least one image sensor (180; 280), and
at least one image fiber (160; 260) arranged between the cascaded interferometer arrangement (120, 140; 220, 240) and the image sensor (180; 280) with respect to the optical path (L_S, L_IL, L_IM) of the OCT device (100; 200; 410), wherein the image fiber (160; 260) is configured to receive at least one interferometric image (L_IM) output by the second interferometer (140; 240) and to transmit the interferometric image (L_IM) towards the image sensor (180; 280) for detection of the interferometric image (L_IM) by means of the image sensor (180; 280).

2. Optical Coherence Tomography, OCT, device according to claim 1, wherein the second interferometer (140; 240) is configured to be arranged in a measuring head (422) of an optical measuring system (400), in particular of an endoscopic measuring system.

3. Optical Coherence Tomography, OCT, device according to claim 1 or claim 2, wherein the light source (110; 210), the first interferometer (120; 220) and the image sensor (180; 280) are configured to be arranged external to the measuring head (422), in particular in a base unit (440) of the optical measuring system.

4. Optical Coherence Tomography, OCT, device according to claim 2 or claim 3, wherein the image fiber (160; 260) is configured to be part of a flexible connection (430) between the measuring head (422) and the image sensor (180; 280).

5. Optical Coherence Tomography, OCT, device according to any one of the preceding claims, further comprising a flexible illumination fiber (230) arranged between the first interferometer (220) and the second interferometer (240).

6. Optical Coherence Tomography, OCT, device according to any one of the preceding claims, wherein the first interferometer (120; 220) is further configured to:
- split light (L_S) received from the light source (110; 210) into at least one primary beam (L_P) and at least one first reference beam (L_R1), and
- output the primary beam (L_P) and the first reference beam (L_R1) at least partially towards the second interferometer (140; 240),
wherein the first interferometer (120; 220) is configured to produce an optical offset (Δ(T)) between the primary beam (L_P) and the first reference beam (L_R1) as output towards the second interferometer (140; 240).

7. Optical Coherence Tomography, OCT, device according to claim 6, wherein the optical offset (Δ(T)) comprises a spatial, a temporal and/or a phase optical offset, in particular wherein the light source (110; 210) and the first interferometer (120; 220) are configured such that the optical offset (Δ(T)) exceeds a coherence length of the light (L_S) received by the first interferometer (120; 220) from the light source (110; 210).

8. Optical Coherence Tomography, OCT, device according to claim 6 or claim 7, wherein the optical offset (Δ(T)) produceable by means of the first interferometer (120; 220) is variable, in particular wherein the OCT device (200; 410) further comprises at least one actuator (228) for varying a position of at least one optical component (226) of the first interferometer (220) for varying the optical offset (Δ(T)).

9. Optical Coherence Tomography, OCT, device according to claim 8, wherein the variable optical offset (Δ(T)) is variable in accordance with a measuring range of the OCT device (200; 410).

10. Optical Coherence Tomography, OCT, device according to any one of the preceding claims, wherein the second interferometer (140; 240) is further configured to:
- split light received from the first interferometer (120; 220) into at least one measuring beam (L_M; L_M1, L_M2) and at least one second reference beam (L_R2);
- output the measuring beam (L_M; L_M1, L_M2) towards a measuring field (M) of the OCT device (100; 200; 410);
- receive at least one object beam (L_O; L_O1, L_O2), the object beam (L_O; L_O1, L_O2) generated by an at least partial reflection of the measuring beam (L_M; L_M1, L_M2) at at least one object (O) in the measuring field (M) of the OCT device (100; 200; 410), and
- output the second reference beam (L_R2) and the object beam (L_O; L_O1, L_O2) at least partially towards the image fiber (160; 260).

11. Optical Coherence Tomography, OCT, device according to claim 10 combined with claim 6, wherein the interferometric image (L_IM) is indicative of at least one depth (D_O; D_OP1, D_OP2) of the object (O) based on a light intensity detectable by means of the image sensor (180; 280), the light intensity produced at least partially by optical interference in accordance with the optical offset (Δ(T)) and the depth (D_O; D_OP1, D_OP2) of the object (O).

12. Optical Coherence Tomography, OCT, device according to claim 11, wherein the optical interference comprises an interference between light of one of the primary beam (L_P) and the first reference beam (L_R1) respectively contained in the second reference beam (L_R2) and light of the respective other one of the primary beam (L_P) and the first reference beam (L_R1) respectively contained in the object beam (L_O; L_O1, L_O2).

13. Optical Coherence Tomography, OCT, device according to any one claims 10 to 12, wherein each one of the second reference beam (L_R2) and the measuring beam (L_M; L_M1, L_M2) output by the second interferometer (140; 240) has a spatially, in particular linearly or planarly, extended cross section for producing a spatially extended interferometric image (L_IM), and the image sensor (180; 280) is configured to provide spatially resolved detection of a light intensity in the interferometric image (L_lM), to enable spatially extended measuring of a depth profile (D_O; D_OP1, D_OP2) of at least one object (O) in the measuring field (M) of the OCT device (100; 200; 410).

14. Optical measuring system (400), in particular an endoscopic measuring system, comprising:
at least one OCT device (410) according to any one of the preceding claims;
at least one measuring head (422), and
at least one base unit (440),
wherein the first interferometer (120; 220; 411), the light source (110; 210; 411) and the image sensor (180; 280; 411) of the OCT device (410) are arranged in the base unit (440), and the second interferometer (140; 240; 413) of the OCT device (410) is arranged in the measuring head (422).

15. Method (500) of operating an optical coherence tomography, OCT, device (100; 200; 410), in particular for an endoscopic measuring system (400), the OCT device (100; 200; 410) comprising:
at least one light source (110; 210);
at least one cascaded interferometer arrangement (120, 140; 220, 240) having at least a first interferometer (120; 220) configured to receive light (L_S) emitted by the light source (110; 210) and at least a second interferometer (140; 240), the second interferometer (140; 240) arranged behind the first interferometer (120; 220) with respect to an optical path (L_S, L_IL, L_IM) of the OCT device (100; 200; 410),
at least one image sensor (180; 280), and
at least one image fiber (160; 260) arranged between the cascaded interferometer arrangement (120, 140; 220, 240) and the image sensor (180; 280) with respect to the optical path (L_S, L_IL, L_IM) of the OCT device (100; 200; 410), wherein the image fiber (160; 260) is configured to receive at least one interferometric image (L_IM) output by the second interferometer (140; 240) and to transmit the interferometric image (L_IM) towards the image sensor (180; 280) for detection of the interferometric image (L_IM) by means of the image sensor (180; 280),
the method (500) comprising:
- outputting (510), by means of a control unit (442) and towards at least one actuator (228) of the OCT device (100; 200; 410), a control signal for varying an optical offset (Δ(T)) between a primary beam (L_P) and a first reference beam (L_R1) as output by the first interferometer (120; 220) of the OCT device (100; 200; 410);
- receiving (520), by means of the control unit (442) and from the image sensor (180; 280) of the OCT device (100; 200; 410), image data of at least one interferometric image (L_IM) for each of various optical offsets (Δ(T));
- analyzing (530), by means of the control unit (442), the image data of the plurality of interferometric images (L_IM) to determine a variable light intensity caused by variable optical interference in accordance with the various optical offsets (Δ(T)), and
- determining (540), by means of the control unit (442), at least one depth (D_O; D_OP1, D_OP2) of at least one object (O) in the measuring field (M) of the OCT device (100; 200; 410) based on the determined variable light intensity.
